# FASCICULE DE BREVET EUROPEEN

(11) **EP 3 157 362 B1**
(45) Date de publication et mention de la délivrance du brevet: **06.02.2019**
(21) Numéro de dépôt: 15726228.8
(22) Date de dépôt: 31.03.2015
(51) Int. Cl.: A24F 47/00, A61M 15/06, A61M 15/00, H05B 1/02

(54) **DISPOSITIF D'AJUSTEMENT D'UNE QUANTITÉ DE SUBSTANCE ACTIVE INHALÉE PAR UN UTILISATEUR ET TERMINAL PORTABLE COMMUNICANT**
VORRICHTUNG ZUR REGELUNG DER MENGE EINES VON EINEM ANWENDER INHALIERTEN WIRKSTOFFS UND TRAGBARES KOMMUNIKATIONSENDGERÄT
DEVICE FOR ADJUSTING AN AMOUNT OF AN ACTIVE SUBSTANCE INHALED BY A USER AND COMMUNICATING PORTABLE TERMINAL

(30) Priorité: 04.04.2014 FR 1400817
(43) Date de publication de la demande: 26.04.2017
(73) Titulaire: ENOVAP, 10430 Rosières-près-Troyes (FR)
(72) Inventeur: SCHECK, Alexandre, 75725 Paris (FR); LEMAIL, Philippe, 75725 Paris (FR); DEVOS, Vincent, 75725 Paris (FR); ELKHOURY, Joseph, 75725 Paris (FR); SUNA, Murat, 75725 Paris (FR); KAYAL, Céline, 75725 Paris (FR)
(74) Mandataire: Cornuejols, Georges
(86) Numéro de dépôt international: PCT/FR2015/050839
(87) Numéro de publication internationale: WO 2015/150699

(56) Documents cités:
- EP-A1- 2 609 820
- WO-A1-2014/004648
- US-A1- 2011 265 806
- US-A1- 2012 048 266
- US-A1- 2012 260 927
- US-A1- 2013 192 615
- US-A1- 2013 284 192

## Description

### DOMAINE TECHNIQUE DE L'INVENTION

La présente invention vise un dispositif d'ajustement d'une quantité de substance active inhalée par un utilisateur et un terminal portable communicant. Elle s'applique, notamment, au sevrage de la nicotine chez un individu fumeur utilisant une cigarette électronique.

### ETAT DE LA TECHNIQUE

L'utilisation d'une cigarette électronique est reconnue comme étant l'un des moyens d'atteindre un arrêt de la dépendance d'un individu à la nicotine. Cependant, dans les systèmes actuels, les cigarettes électroniques sont susceptibles de remplacer les cigarettes classiques du fait de l'absence de sevrage délivré par ces systèmes.

Dans les systèmes actuels, le sevrage en nicotine d'un individu grâce à une cigarette électronique est basé uniquement sur la capacité de cet individu à se discipliner. Il est bien connu, qu'en raison des mécanismes de la dépendance à la nicotine, la volonté seule de l'individu est fréquemment insuffisante dans la durée.

On connaît, en particulier, des systèmes tels que décrits dans le document US 2011/265806. Ces systèmes prévoient un dispositif d'ajustement d'une quantité de nicotine inhalée par un utilisateur comportant deux réservoirs remplis chacun par un liquide présentant une quantité de nicotine différente et un moyen d'inhalation de la vapeur issues de ces réservoirs. Toutefois, ces systèmes ne permettent aucun ajustement de la quantité de nicotine inhalée par l'utilisateur.

On connait également une cigarette électronique à double réservoir telle que décrite dans la demande de brevet américain US 2013/284192. Ce document divulgue un dispositif avec les caractéristiques du préambule de la revendication 1.

### OBJET DE L'INVENTION

La présente invention vise à remédier à tout ou partie de ces inconvénients.

A cet effet, selon un premier aspect, la présente invention vise un dispositif d'ajustement d'une quantité de substance active inhalée par un utilisateur, qui comporte :
- deux réservoirs, un premier réservoir comportant un liquide présentant une densité de substance active moins élevée qu'un liquide dans le deuxième réservoir, chaque liquide étant configuré pour s'évaporer lorsque ce liquide est chauffé au delà d'une température limite prédéterminée,
- un moyen d'inhalation, par l'utilisateur, de la vapeur de liquide s'évaporant, en provenance de chaque réservoir,
- deux résistances chauffantes, chaque réservoir étant associé à une résistance chauffante,
- un moyen de détermination d'une quantité de substance active à vaporiser pour un volume de vapeur prédéterminé,
- un moyen de commande de l'échauffement de chaque résistance pour actionner indépendamment chaque résistance en fonction de la quantité de substance active à vaporiser déterminée, le ratio de l'échauffement de la résistance associée au deuxième réservoir sur l'échauffement de la résistance associée au premier réservoir étant une fonction croissante de la quantité de substance active à vaporiser déterminée pour le volume de vapeur prédéterminé.

Grâce à ces dispositions, la quantité de substance active inhalée par un utilisateur du dispositif est modulée par l'échauffement du premier ou du deuxième liquide, chacun de ces liquides présentant une densité de substance active différente. De cette manière, à quantité de vapeur inhalée identique, à chaque inhalation, la part de substance active dans la vapeur inhalée est différente à chaque inhalation.

Dans des modes de réalisation, le dispositif objet de la présente invention comporte un moyen d'accès à un profil d'utilisateur, le moyen de détermination d'une quantité de substance active à vaporiser déterminant la quantité en fonction de données du profil d'utilisateur.

L'avantage de ces modes de réalisation est qu'ils permettent d'adapter la quantité de substance active à un profil type d'utilisateur ou à une donnée particulière d'un utilisateur ayant renseigné une information morphologique et/ou médicale dans un profil.

Dans des modes de réalisation, le moyen de détermination détermine une quantité de substance active à vaporiser en fonction d'une donnée d'horodatage associée à une mise en marche du dispositif.

Ces modes de réalisation ont l'avantage de permettre d'adapter la quantité de substance active à un instant de la journée ou de la semaine.

Dans des modes de réalisation, le moyen de détermination détermine une quantité de substance active croissante, par rapport la dernière quantité de substance active déterminée, lorsque la donnée d'horodatage est la première donnée d'horodatage supérieure à une heure prédéterminée.

L'avantage de ces modes de réalisation est qu'ils permettent, le matin par exemple, d'augmenter la quantité de substance active inhalée.

Dans des modes de réalisation, le moyen de détermination détermine une quantité de substance active globalement décroissante en fonction de la donnée d'horodatage.

Ces modes de réalisation ont l'avantage de permettre un sevrage de l'utilisateur.

Dans des modes de réalisation, le dispositif objet de la présente invention comporte un moyen de détection d'une fréquence d'inhalation de l'utilisateur sur le moyen d'inhalation, le moyen de détermination déterminant la quantité de substance active en fonction de la fréquence d'inhalation détectée.

L'avantage de ces modes de réalisation est de permettre, lorsque l'utilisateur présente un manque marqué de substance active, comme par exemple au cours d'une soirée, d'augmenter la quantité de substance active inhalée.

Dans des modes de réalisation, le dispositif objet de la présente invention comporte un moyen de capture d'une alcoolémie de l'utilisateur, le moyen de détermination déterminant la quantité de substance active à vaporiser en fonction de l'alcoolémie captée.

Ces modes de réalisation ont l'avantage, lorsqu'une alcoolémie détectée est inférieure à une valeur limite prédéterminée, de permettre d'augmenter ou de réduire la concentration de substance active par l'inhalation.

Dans des modes de réalisation, le dispositif comporte un moyen de capture d'une donnée représentative d'une température dans au moins un réservoir, le moyen de commande commandant l'échauffement de la résistance associée à chaque dit réservoir en fonction de la température captée.

L'avantage de ces modes de réalisation est qu'ils permettent de détecter un dysfonctionnement d'une résistance par exemple. De plus, ces modes de réalisation permettent de prendre en compte le temps de refroidissement caractéristiques de chaque résistance.

Selon un deuxième aspect, la présente invention vise un ensemble comportant un dispositif objet de la présente invention, et un terminal portable communicant, ledit terminal comportant :
- un moyen de détermination d'une information représentative d'une quantité de substance active à vaporiser et
- un moyen de transmission, à un dispositif objet de la présente invention, de l'information représentative d'une quantité de substance active à vaporiser objet de la présente invention.

Les avantages, buts et caractéristiques particulières de ce terminal portable communicant étant similaires à ceux du dispositif d'ajustement d'une quantité de substance active à inhaler objet de la présente invention, ils ne sont pas rappelés ici.

### BREVE DESCRIPTION DES FIGURES

D'autres avantages, buts et caractéristiques particulières de l'invention ressortiront de la description non limitative qui suit d'au moins un mode de réalisation particulier du dispositif et du terminal objets de la présente invention, en regard des dessins annexés, dans lesquels :
- la figure 1 représente, schématiquement et en coupe, un premier mode de réalisation particulier du dispositif objet de la présente invention,
- la figure 2 représente, schématiquement et en coupe, un mode de réalisation particulier du terminal objet de la présente invention,
- la figure 3 représente, schématiquement et en coupe, un logigramme d'étapes particulier du procédé objet de la présente invention,
- la figure 4 représente, schématiquement et en coupe, un deuxième mode de réalisation particulier du dispositif objet de la présente invention et
- la figure 5 représente, schématiquement et en coupe, un troisième mode de réalisation particulier du dispositif objet de la présente invention.

### DESCRIPTION D'EXEMPLES DE REALISATION DE L'INVENTION

La présente description est donnée à titre non limitatif, chaque caractéristique d'un mode de réalisation pouvant être combinée à toute autre caractéristique de tout autre mode de réalisation de manière avantageuse. Par ailleurs, chaque paramètre d'un exemple de réalisation peut être mis en oeuvre indépendamment d'autres paramètres dudit exemple de réalisation.

On note que le terme « un » est utilisé au sens de « au moins un ».

On note dès à présent que les figures ne sont pas à l'échelle.

On note, dès à présent, que le terme « substance active » se réfère, à titre non limitatif, à tout principe actif thérapeutique et à la nicotine par exemple.

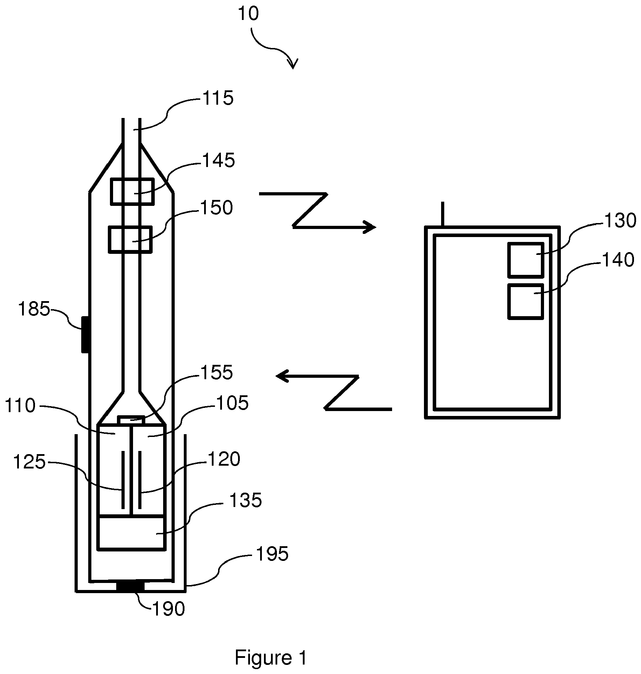

On observe, sur la figure 1, qui n'est pas à l'échelle, une vue en coupe d'un mode de réalisation du dispositif 10 objet de la présente invention. Ce dispositif 10 comporte :
- deux réservoirs, 105 et 110, un premier réservoir 105 comportant un liquide présentant une densité de substance active moins élevée qu'un liquide dans le deuxième réservoir 110, chaque liquide étant configuré pour s'évaporer lorsque ce liquide est chauffé au delà d'une température limite prédéterminée,
- un moyen 115 d'inhalation, par l'utilisateur, de la vapeur de liquide s'évaporant, en provenance de chaque réservoir, 105 et 110,
- deux résistances, 120 et 125, chauffantes, chaque réservoir, 105 et 110, étant associé à une résistance chauffante, 120 et 125,
- un moyen 130 de détermination d'une quantité de substance active à vaporiser,
- un moyen 135 de commande de l'échauffement de chaque résistance, 120 et 125,
- un moyen 140 d'accès à un profil d'utilisateur,
- un moyen 145 de détection d'une fréquence d'inhalation de l'utilisateur sur le moyen 115 d'inhalation,
- un moyen 150 de capture d'une alcoolémie de l'utilisateur et
- un moyen 155 de capture d'une donnée représentative d'une température dans au moins un réservoir, 105 et 110.

Les deux réservoirs, 105 et 110, sont, par exemple deux réservoirs de dimensions identiques configurés pour être transportables dans un dispositif de dimensions comparables à celles d'une cigarette électronique. Ces réservoirs, 105 et 110, par exemple, présentent des dimensions pour contenir 6mL de liquide chacun. Chacun de ces réservoirs, 105 et 110, comporte une cavité, non représentée, permettant l'insertion d'une résistance chauffante, 120 et 125. Une résistance chauffante, 120 ou 125, est associé à chaque réservoir, 105 ou 110, de manière ce que lorsqu'une résistance chauffante, 120 ou 125, est échauffée, seul le liquide contenu dans le réservoir, 105 ou 110, associé soit échauffé.

Dans des variantes, les deux réservoirs, 105 et 110, sont positionnés de manière parallèle le long d'un axe longitudinal d'un tube globalement en forme de cylindre de révolution. Ce tube comporte, en aval d'un côté du chemin d'air passant par une sortie de chaque réservoir, le moyen d'inhalation 115 et, en amont du chemin d'air, une entrée d'air, non représentée.

Dans des variantes, le dispositif 10 comporte au moins trois réservoirs.

Le moyen d'inhalation 115 est, par exemple, un conduit configuré pour permettre à un utilisateur d'inhaler les vapeurs en sortie des réservoirs, 105 et 110.

Les deux résistances, 120 et 125, sont, par exemple, des résistances électriques s'échauffant par effet Joule lorsqu'un courant est appliqué à ces résistances. L'échauffement d'une telle résistance, 120 et 125, dépend de l'intensité du courant traversant ladite résistance, 120 et 125. Ainsi, l'échauffement de la résistance, 120 et 125, peut être modulé par un moyen de commande 135 configuré pour appliquer un courant d'une intensité déterminée à cette résistance, 120 et 125.

Le moyen de détermination 130 est, par exemple, un programme informatique intégré à un terminal portable communicant et/ou du dispositif comportant les réservoirs, 105 et 110. Ce terminal portable communicant est, par exemple, un téléphone mobile intelligent ou une tablette numérique. Ce moyen de détermination 130 comporte un moyen d'accès 140 à un profil d'utilisateur. Ce profil d'utilisateur correspond à un profil d'utilisateur type déterminé en fonction de données de consommation déclarées ou apprises de l'utilisateur. Ces données de consommation comportent, par exemple :
- une fréquence de consommation en fonction d'un moment d'une journée ou d'une semaine,
- un moment d'une journée de consommation habituel et
- répartition de l'inhalation de substance active provenant d'une cigarette électronique et d'une cigarette.

Dans le cas où les données de consommation sont apprises, ces données sont obtenues par accumulation de données associées à l'utilisation du moyen de commande mémorisées. Le moyen de commande est, par exemple, pendant une période d'apprentissage, configuré pour commander la vaporisation d'une quantité constante de substance active. Chaque inhalation est datée par un moyen d'horodatage, tel une horloge électronique. Des données représentatives de chaque inhalation sont transmises par le biais d'un moyen de transmission à une mémoire. Ce moyen de transmission est, par exemple, une antenne configurée pour émettre un signal sans fil selon la technologie Bluetooth. Dans des variantes, la mémoire est dans un même boîtier que le moyen d'inhalation. Dans d'autres variantes, la mémoire est intégrée au terminal portable communicant. Dans d'autres variantes, la mémoire est distante.

En fonction des données mémorisées, un moyen de détermination d'un profil d'utilisateur détermine un profil d'utilisateur. Ce moyen de détermination d'un profil d'utilisateur est, par exemple, un programme informatique configuré pour comparer une courbe de consommation de substance active en fonction du temps à l'échelle d'une journée et/ou d'une semaine à des courbes types de consommation. Lorsqu'une courbe type de consommation la plus proche de la courbe de consommation apprise est déterminée, le moyen de détermination d'un profil d'utilisateur détermine que le profil d'utilisateur associé à cette courbe type correspond au profil type de l'utilisateur dont le mode de consommation a été appris.

Le moyen d'accès 140 est, par exemple, une antenne configurée pour communiquer avec un serveur distant comportant des données relatives au profil d'utilisateur.

Le moyen de détermination 130 est configuré pour déterminer une quantité de substance active à vaporiser en fonction d'une courbe de sevrage type associée au profil d'utilisateur type déterminé. Cette courbe est globalement décroissante en fonction du temps à une échelle d'une semaine par exemple. Cependant cette courbe peut être croissante à certains moments d'une journée ou d'une semaine en fonction des habitudes constatées de consommation de l'utilisateur.

Une donnée d'horodatage est associée à un instant de détermination par le moyen de détermination 130. Cette donnée d'horodatage est obtenue, par exemple, par une horloge électronique configurée pour mesurer une date et une heure d'activation d'un des moyens du dispositif 10.

Le moyen de détermination 130 d'une quantité de substance active à vaporiser déterminant la quantité en fonction de données du profil d'utilisateur.

Le moyen de détermination 130 détermine une quantité de substance active à vaporiser en fonction d'une donnée d'horodatage associée à une mise en marche du dispositif 10.

Le moyen de détermination 130 détermine une quantité de substance active croissante, par rapport la dernière quantité de substance active déterminée, lorsque la donnée d'horodatage est la première donnée d'horodatage supérieure à une heure prédéterminée. Par exemple, la première inhalation de la journée présente une quantité de substance active plus grande que la dernière inhalation de la journée précédente.

Dans des variantes, le moyen de détermination 130 détermine une quantité de substance active croissante lorsqu'une durée supérieure à une durée limite prédéterminée s'est écoulée depuis la dernière inhalation.

Le moyen de détermination 130 détermine une quantité de substance active globalement décroissante en fonction de la donnée d'horodatage.

Le moyen de détection 145 d'une fréquence d'inhalation de l'utilisateur sur le moyen d'inhalation 115 est, par exemple, un circuit électronique comportant un compteur du nombre d'inhalations réalisées par l'utilisateur sur le moyen d'inhalation 115. Le nombre d'inhalation est déterminé, par exemple, par l'utilisation d'une hélice configurée pour tourner lorsque de l'air traverse le conduit du moyen d'inhalation 115 dans un sens prédéterminé. Ce nombre d'inhalation, mesuré pendant une durée limite prédéterminée glissante, divisé par la durée limite prédéterminée glissante, donne une fréquence d'inhalation.

Lorsque cette fréquence d'inhalation est supérieure à une fréquence limite prédéterminée, le moyen de détermination 130 détermine une quantité de substance active à vaporiser croissante par rapport à la précédente quantité de substance active déterminée. De manière générale, le moyen de détermination 130 détermine la quantité de substance active en fonction de la fréquence d'inhalation détectée.

Le dispositif 10 comporte un moyen de capture 150 d'une alcoolémie de l'utilisateur. Ce moyen de capture 150 est, par exemple, un éthylotest associé au moyen d'inhalation 115.

Le moyen de détermination 130 détermine la quantité de substance active à vaporiser en fonction de l'alcoolémie captée. Si l'alcoolémie captée est élevée, et qu'une donnée, variable ou non, du profil d'utilisateur indique que l'utilisateur est conducteur, la quantité de substance active déterminée est augmentée. Inversement, si le profil d'utilisateur indique que l'utilisateur n'est pas conducteur, la quantité de substance active déterminée est réduite.

Dans des variantes, le moyen de détermination 130 est intégré au même boîtier que le moyen d'inhalation 115. Dans d'autres variantes, le moyen de détermination 130 est dans une mémoire distante, telle un serveur par exemple.

La quantité de substance active à vaporiser déterminée est émise, par un moyen d'émission d'une information représentative de la quantité de substance active déterminée, en direction du moyen de commande 135. Ce moyen d'émission est, par exemple, une antenne du terminal portable communicant comportant le moyen de détermination 130 configurée pour émettre un signal selon la technologie Bluetooth.

Le moyen de commande 135 est, par exemple, un microcontrôleur connecté à une source d'alimentation autonome, non représentée. Le moyen de commande 135 transmet à chaque résistance, 120 et 125, un courant électrique en fonction de la quantité de substance active à vaporiser déterminée.

Dans des variantes, la source d'alimentation autonome est rechargée par une dynamo positionnée sur le chemin d'air à l'intérieur du conduit et actionnée par le passage de l'air grâce à une hélice par exemple.

Le moyen de commande 135 de l'échauffement de chaque résistance, 120 et 125, pour actionner indépendamment chaque résistance, 120 et 125, en fonction de la quantité de substance active à vaporiser déterminée, le ratio de l'échauffement de la résistance 125 associée au deuxième réservoir 110 sur l'échauffement de la résistance 120 associée au premier réservoir 105 étant une fonction croissante de la quantité de substance active à vaporiser déterminée.

Ce moyen de commande 135 transmet un courant électrique en fonction des liquides présents dans chaque réservoir, 105 et 110. La nature de ces liquides peut être transmise par le terminal portable communicant.

Dans des variantes, le dispositif 10 comporte un bouton présentant deux états. Dans un premier état, le moyen de commande 135 est inhibé et aucune régulation de la quantité de substance active n'a lieu. Dans un deuxième état, le moyen de commande 135 fonctionne de la manière décrite ci-dessus. Ce bouton peut être remplacé par une antenne de réception d'une commande contenue dans un signal sans-fil émis, par exemple, par le terminal portable communicant.

Dans des variantes, le dispositif 10 comporte une bague rotative dont le degré de rotation détermine une quantité de substance active à vaporiser ou un ratio d'échauffement des deux résistances, 120 et 125.

Dans des variantes, le dispositif 10 comporte une bague rotative dont le degré de rotation détermine une puissance globale d'échauffement des deux résistances, 120 et 125.

Dans des variantes, les bagues rotatives sont confondues et un bouton (non représenté) permet le passage de la détermination de la puissance globale à la détermination de la quantité de substance. Ce bouton peut être remplacé par une antenne de réception d'une commande contenue dans un signal sans-fil émis, par exemple, par le terminal portable communicant.

Dans des variantes, la bague rotative ou le bouton est remplacé par une zone tactile 185, un mouvement de doigt de l'utilisateur sur cette zone tactile permettant de régler le niveau de substance active à évaporer. Par exemple :
- si l'utilisateur balaie la zone tactile 185 vers un embout d'aspiration, la quantité de nicotine à évaporer est augmentée,
- si l'utilisateur balaie la zone tactile 185 vers une extrémité opposée à l'embout d'aspiration, la quantité de nicotine à évaporer est réduite,
- si l'utilisateur balaie la zone tactile 185 vers un premier côté du dispositif 10, le ratio de puissance d'échauffement d'une première résistance par rapport à la puissance d'échauffement de la deuxième résistance augmente et
- si l'utilisateur balaie la zone tactile 185 vers un deuxième côté du dispositif 10, le ratio de puissance d'échauffement de la première résistance par rapport à la puissance d'échauffement de la deuxième résistance diminue.

Le dispositif 10 comporte un moyen de capture 155 d'une donnée représentative d'une température dans au moins un réservoir, 105 et 110. Ce moyen de capture 155 est, par exemple, un thermomètre électronique.

Le moyen de commande 135 commande l'échauffement de la résistance, 120 et 125, associée à chaque dit réservoir, 105 et 110, en fonction de la température captée.

Dans des variantes, le dispositif 10 comporte un moyen de capture du débit d'inhalation d'un utilisateur. Ce moyen de capture du débit est, par exemple, un circuit électronique connecté à une hélice positionnée dans le conduit. En fonction d'une rotation de l'hélice captée et d'une valeur prédéterminée représentative de la surface de la section du conduit à l'endroit de l'hélice, le moyen de capture du débit calcule le débit d'inhalation.

Dans des variantes, le dispositif 10 comporte un moyen d'émission à une mémoire distante d'information de consommation de l'utilisateur. Ce moyen d'émission est, par exemple, une antenne configurée pour émettre un signal sans fil selon le standard IEEE 802.11, dit « Wi-Fi », par exemple. Les informations de consommation ainsi mémorisées permettent, par exemple, d'établir des statistiques transmises à un terminal portable communicant de l'utilisateur.

Dans des variantes, le dispositif 10 comporte une housse 195 de protection des réservoirs, 105 et 110, détachable, cette housse 195 comportant un moyen 190 de recharge des systèmes électriques du dispositif 10. Ce moyen de recharge 190 est, par exemple, une tige conductrice d'électricité, de type micro-USB par exemple, mise en contact d'une tige d'alimentation (non représentée) du dispositif 10. Dans des variantes, ce moyen de recharge 190 met en oeuvre une recharge à induction. Cette housse 195 comporte, par exemple, une alimentation en électricité, telle une pile ou une batterie par exemple.

Dans des variantes, le dispositif 10 comporte un écran d'affichage d'informations représentatives :
- d'un niveau de charge de la batterie,
- d'un niveau de remplissage d'un ou de chaque réservoir,
- d'un mode de consommation, automatique ou manuel, de la substance active et/ou
- d'un ratio d'échauffement entre les deux résistances,
- d'une valeur des résistances détectées en Ohm,
- d'une usure des résistances en pourcentage,
- d'une température en temps réel des résistances,
- d'une puissance totale ou tension totale aux bornes de chaque résistance,
- d'une valeur du ratio de puissance d'échauffement entre les résistances, et/ou
- de divers messages sous forme de texte.

Dans des variantes, le dispositif 10 comporte un moyen d'émission d'un signal lumineux. Ce moyen d'émission d'un signal lumineux est, par exemple, une diode électroluminescente configurée pour émettre de la lumière lorsqu'une fréquence d'inhalation de l'utilisateur détectée est supérieure à une valeur limite prédéterminée.

Dans des variantes, le moyen de commande 135 est désactivé pendant une durée limite prédéterminée lorsqu'une quantité limite prédéterminée de substance active a été vaporisée pendant une durée limite prédéterminée.

Dans des variantes, au moins un des réservoirs, 105 et 110, comporte un médicament configuré pour être pris par voie orale ou inhalatoire. Ce médicament est, par exemple, sous forme de grandes molécules brisées par un moyen d'émission d'ultrasons.

Dans des variantes, le moyen de détermination 130 détermine une quantité de substance active à inhaler en fonction d'une information d'un événement, déclarée par l'utilisateur, associée à une donnée d'horodatage. Lorsque la détermination d'une quantité de substance active a lieu pendant l'événement mémorisé, la quantité de substance active déterminée est augmentée.

Dans des variantes, le moyen d'inhalation 115 est connecté à un moyen de géolocalisation et une donnée représentative d'une localisation est associée en mémoire à chaque donnée d'une inhalation.

Dans des variantes, au moins un moyen d'émission émettant un signal selon la technologie Bluetooth met en oeuvre la technologie Bluetooth Low Energy (traduite par « Bluetooth à faible consommation énergétique » en français).

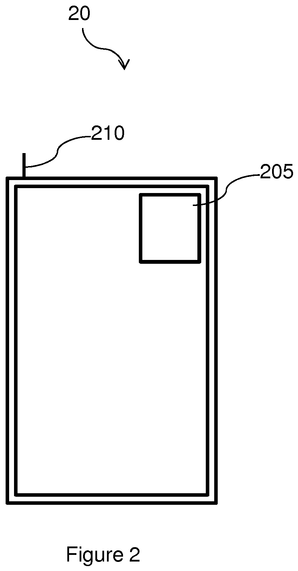

On observe, sur la figure 2, un mode de réalisation particulier du terminal 20 portable communicant. Ce terminal 20 comporte :
- un moyen 205 de détermination d'une information représentative d'une quantité de substance active à vaporiser et
- un moyen 210 de transmission, à un dispositif 10, tel que décrit en figure 1, de l'information représentative d'une quantité de substance active à vaporiser.

Le moyen de détermination 205 est similaire au moyen de détermination 130 décrit en figure 1.

Le moyen de transmission 210 est, par exemple, une antenne configurée pour émettre un signal sans fil selon la technologie Bluetooth.

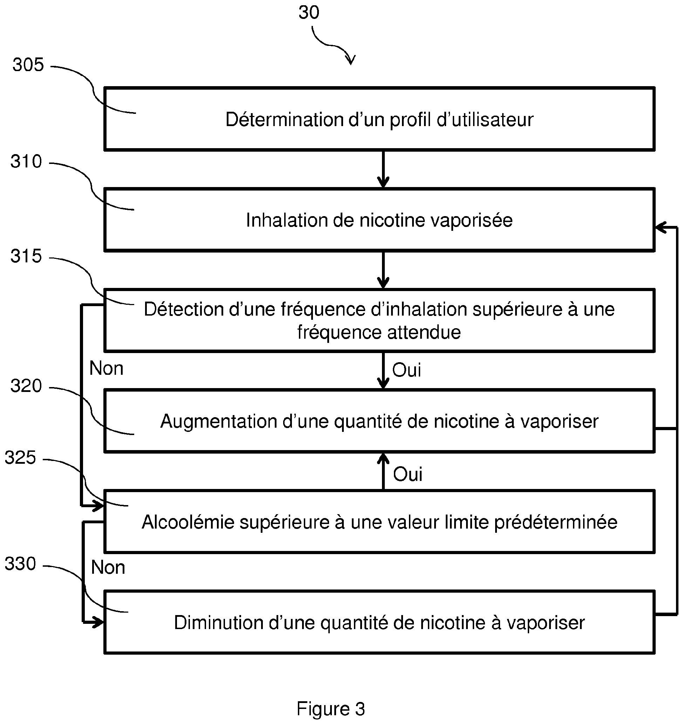

On observe, sur la figure 3, un logigramme d'étape particulier du procédé 30 objet de la présente invention. Ce procédé 30 comporte une étape 305 de détermination d'un profil d'utilisateur et, de manière itérative :
- une étape 310 d'inhalation d'une quantité de substance active vaporisée,
- une étape 315 de détection d'une fréquence d'inhalation supérieure à une fréquence attendue,
- une étape 320 d'augmentation de la quantité de substance active à vaporiser,
- une étape 325 de détection d'une alcoolémie d'un utilisateur,
- une étape 330 de diminution d'une quantité de substance active à vaporiser.

Lorsqu'un utilisateur muni du dispositif 10 tel que décrit en figure 1 démarre un sevrage de la substance active, cet utilisateur est associé à un profil d'utilisateur. Ce profil d'utilisateur est soit déclaratif, soit appris.

Dans les variantes où ce profil d'utilisateur est déclaratif, l'utilisateur renseigne, grâce à une interface utilisateur, telle une application d'un terminal portable communicant par exemple, un nombre de champs d'information prédéterminé. Ces champs d'information comportent, par exemple, des informations concernant un mode de consommation de substance active, par exemple en termes de :
- fréquence,
- moment de consommation et
- répartition entre cigarette électronique et cigarette.

D'autres champs d'information peuvent comporter des informations relatives à des données morphologiques ou médicales de l'utilisateur.

Dans les variantes où ce profil est appris, le dispositif 10 mémorise le comportement d'un utilisateur, en fonction de données d'horodatage, sans ajuster une quantité de substance active vaporisée lors d'une inhalation de vapeurs de l'utilisateur. Les données ainsi mémorisées permettent, par exemple, de déterminer les instants de la journée où l'utilisateur inhale le plus fréquemment de la substance active ainsi qu'un schéma habituel de consommation en fonction du jour de la semaine. Par exemple, un utilisateur peut consommer plus de substance active le vendredi soir que le mardi après-midi. Dans ces variantes, l'apprentissage réalisé peut continuer une fois que le moyen de détermination commence à déterminer une quantité de substance active à vaporiser.

Ce profil d'utilisateur déclaratif ou appris est associé, dans des variantes, à un profil d'utilisateur type prédéterminé. Ce profil d'utilisateur type représente globalement un certain mode de consommation de substance active par un utilisateur. Le sevrage réalisé dépend, comme décrit ci-dessous, de ce profil d'utilisateur type associé au profil d'utilisateur.

Ce profil d'utilisateur est stocké, par exemple, dans une mémoire du terminal portable communicant. Ce terminal portable communicant est, par exemple, un téléphone mobile intelligent ou une tablette numérique. Dans des variantes, ce profil d'utilisateur est stocké dans une mémoire associée au moyen de détermination du dispositif 10. Dans d'autres variantes, ce profil d'utilisateur est stocké dans une mémoire distante associée au dispositif 10 et dont le moyen de détermination peut obtenir, via une transmission de signal sans-fil, des données du profil stocké.

Lorsque le profil d'utilisateur a été déterminé, le moyen de détermination du dispositif 10 détermine une quantité de substance active initiale à vaporiser comparable à une quantité de substance active habituellement consommée par l'utilisateur en fonction du profil type d'utilisateur associé à l'utilisateur.

Puis, chaque nouvelle détermination d'une quantité de substance active à vaporiser est globalement inférieure aux quantités déterminées précédemment de manière à suivre une courbe de sevrage type associée au profil type. Cette courbe, bien que globalement décroissante, peut comporter des pics de substance active temporaires certains jours de la semaine par exemple. Le moyen de détermination du dispositif 10 détermine la quantité de substance active à vaporiser en fonction d'une mémoire des quantités déterminées précédemment et d'un horodatage capté.

Lorsqu'une fréquence d'inhalation, par un utilisateur, sur le moyen d'inhalation du dispositif 10 est supérieure à une fréquence limite prédéterminée, le moyen de détermination est configuré pour déterminer une quantité de substance active supérieure à la quantité de substance active déterminée par l'utilisation de la courbe type.

Lorsqu'une alcoolémie d'un utilisateur du dispositif 10 captée est supérieure à une valeur limite prédéterminée, la quantité de substance active à vaporiser déterminée est supérieure à la quantité de substance active déterminée par l'utilisation de la courbe type. La substance active agit, contrairement à l'alcool, comme un moyen de concentration de l'utilisateur. Cette concentration, lorsque l'utilisateur conduit un véhicule par exemple, permet d'éviter des risques d'accidents de la route.

Dans des variantes, l'alcoolémie captée n'est considérée que si l'utilisateur a déclaré, dans le profil d'utilisateur, être conducteur. Dans des variantes, le dispositif 10 comporte un moyen de commutation entre une déclaration, de la part de l'utilisateur, du statut de conducteur et du statut de non-conducteur. Ce moyen de commutation est, par exemple, un bouton d'une interface graphique du terminal portable communicant du dispositif 10. En fonction du statut sélectionné, le moyen de détermination du dispositif 10 détermine la quantité de substance active tel que décrit ci-dessus ou non.

Quand une quantité de substance active à vaporiser a été déterminée, une information représentative de cette quantité de substance active déterminée est transmise à un moyen de commande du dispositif 10. Ce moyen de commande est configuré pour actionner l'échauffement d'une première et/ou d'une deuxième résistance associée, chacune, à un réservoir comportant un liquide. Un premier réservoir comporte un liquide présentant une densité de substance active moindre par rapport au liquide dans le deuxième réservoir. Ce moyen de commande est, par exemple, un circuit électronique connecté à un moyen de réception de l'information représentative d'une quantité de substance active à vaporiser. Ce moyen de réception est, par exemple, une antenne configurée pour recevoir un signal sans fil émis par un moyen d'émission associé au moyen de détermination.

Dans des variantes, l'un des réservoirs comporte un liquide dont un élément vaporisé réagit avec la nicotine contenue dans une cigarette pour produire un goût et/ou une odeur désagréable.

Dans des variantes, chaque réservoir comporte un liquide présentant un goût différent, le dispositif 10 visant à permettre un ajustement du goût de la vapeur. Cet ajustement peut être obtenu par l'utilisation simultanée des deux réservoirs, soit l'un après l'autre.

En fonction de l'information de la quantité de substance active déterminée reçue, le moyen de commande du dispositif 10 commande l'actionnement de la première et/ou de la deuxième résistance de manière à ce que le volume de vapeur soit identique, entre deux inhalations, et la quantité de substance active dans ce volume différente. Lorsqu'une plus grande quantité de vapeur du premier liquide, présentant une densité de substance active plus faible, est vaporisée, la vapeur totale formée par la somme des vapeurs de chaque réservoir présente une quantité de substance active diminuée par rapport à une vapeur uniquement composée de vapeur issue du deuxième réservoir.

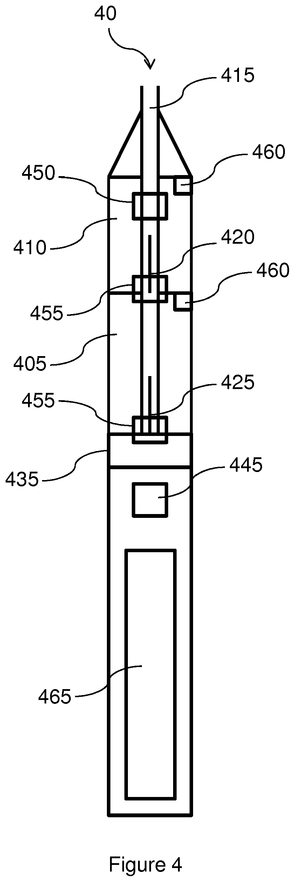

On observe, sur la figure 4, un mode de réalisation particulier du dispositif 40 objet de la présente invention. Les références 405, 410, 415, 420, 425, 435, 445, 450 et 455 correspondent, par exemple, aux références 405, 110, 115, 120, 125, 135, 145, 150 et 155 décrites en regard de la figure 1.

Dans ce mode de réalisation, les réservoirs, 405 et 410, sont agencés en étages, une résistance 420 et 425, étant positionnée dans chaque réservoir, 405 et 410. Dans ce mode de réalisation, le dispositif 40 comporte un moyen de capture 455 de la température pour chaque réservoir, 405 et 410.

Dans ce mode de réalisation, le dispositif 40 comporte deux éléments détachables. Un élément, dit « inférieur », comporte une batterie 165 et le moyen de détection 145.

Dans ce mode de réalisation, chaque réservoir, 105 et 110, comporte une valve étanche d'injection de liquide permettant le passage de liquide dans un sens unique vers l'intérieur dudit réservoir, 105 ou 110. Cette valve permet, notamment, l'insertion d'un embout de fiole de liquide pour cigarette électronique.

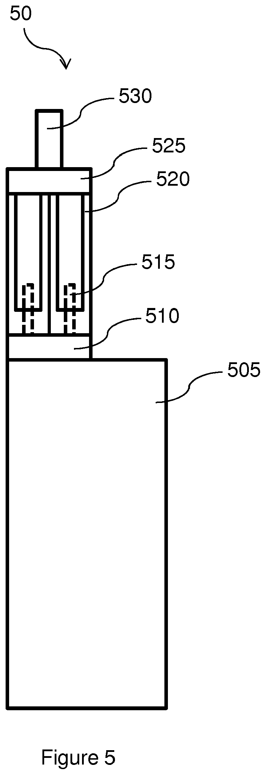

On observe, sur la figure 5, un troisième mode de réalisation particulier du dispositif 50 objet de la présente invention. Ce dispositif 50 comporte :
- un boîtier 505 comportant une batterie (non représentée) d'alimentation des systèmes électriques du dispositif 50,
- une bague 510 de connexion vissée sur le boîtier 505,
- un réservoir 520 comportant plusieurs compartiments,
- pour chaque compartiment, un atomiseur 515 vissé à la bague 510 et encastré dans le réservoir 520 en regard du compartiment correspondant,
- une chambre 525 de mélange des vapeurs fixée au réservoir 520, cette chambre comportant, dans des variantes, des valves (non représentées) d'injection de liquide dans les compartiments et
- un embout 530 d'aspiration des vapeurs.

Dans des modes de réalisation (non représentés), la présente invention vise un dispositif similaire au dispositif 10 ou au dispositif 40 adapté pour la consommation de morphine en guise de substance active.

Dans des modes de réalisation (non représentés), la présente invention vise un dispositif similaire au dispositif 10 ou au dispositif 40 adapté pour la consommation de THC en guise de substance active.

Dans des modes de réalisation (non représentés), la présente invention vise un dispositif similaire au dispositif 10 ou au dispositif 40 adapté pour la consommation d'huile essentielle en guise de substance active.

## Revendications

1. Dispositif (10) d'ajustement d'une quantité de substance active inhalée par un utilisateur, qui comporte :
- deux réservoirs (105, 110), un premier réservoir (105) comportant un liquide présentant une densité de substance active moins élevée qu'un liquide dans le deuxième réservoir (110), chaque liquide étant configuré pour s'évaporer lorsque ce liquide est chauffé au delà d'une température limite prédéterminée,
- un moyen (115) d'inhalation, par l'utilisateur, de la vapeur de liquide s'évaporant, en provenance de chaque réservoir,
- deux résistances (120, 125) chauffantes, chaque réservoir étant associé à une résistance chauffante,
**caractérisé en ce qu'**il comporte :
- un moyen (130) de détermination d'une quantité de substance active à vaporiser pour un volume de vapeur prédéterminé,
- un moyen (135) de commande de l'échauffement de chaque résistance pour actionner indépendamment chaque résistance en fonction de la quantité de substance active à vaporiser déterminée, le ratio de l'échauffement de la résistance associée au deuxième réservoir sur l'échauffement de la résistance associée au premier réservoir étant une fonction croissante de la quantité de substance active à vaporiser déterminée pour le volume de vapeur prédéterminé.

2. Dispositif (10) selon la revendication 1, qui comporte un moyen (140) d'accès à un profil d'utilisateur,
le moyen (130) de détermination d'une quantité de substance active à vaporiser déterminant la quantité en fonction de données du profil d'utilisateur.

3. Dispositif (10) selon l'une des revendications 1 ou 2, dans lequel le moyen (130) de détermination détermine une quantité de substance active à vaporiser en fonction d'une donnée d'horodatage associée à une mise en marche du dispositif.

4. Dispositif (10) selon la revendication 3, dans lequel le moyen (130) de détermination détermine une quantité de substance active croissante, par rapport la dernière quantité de substance active déterminée, lorsque la donnée d'horodatage est la première donnée d'horodatage supérieure à une heure prédéterminée.

5. Dispositif (10) selon la revendication 3 ou 4, dans laquelle le moyen (130) de détermination détermine une quantité de substance active globalement décroissante en fonction de la donnée d'horodatage.

6. Dispositif (10) selon l'une des revendications 3 à 5, qui comporte un moyen (145) de détection d'une fréquence d'inhalation de l'utilisateur sur le moyen (115) d'inhalation, le moyen (130) de détermination déterminant la quantité de substance active en fonction de la fréquence d'inhalation détectée.

7. Dispositif (10) selon l'une des revendications 1 à 6, qui comporte un moyen (150) de capture d'une alcoolémie de l'utilisateur, le moyen (130) de détermination déterminant la quantité de substance active à vaporiser en fonction de l'alcoolémie captée.

8. Dispositif (10) selon l'une des revendications 1 à 7, qui comporte un moyen (155) de capture d'une donnée représentative d'une température dans au moins un réservoir (105, 110), le moyen (135) de commande commandant l'échauffement de la résistance (120, 125) associée à chaque dit réservoir en fonction de la température captée.

9. Ensemble comportant un dispositif selon l'une des revendications 1 et 8, et un terminal (20) portable communicant, **caractérisé en ce que** le terminal comporte :
- un moyen (205) de détermination d'une information représentative d'une quantité de substance active à vaporiser et
- un moyen (210) de transmission, à un dispositif (10) selon l'une des revendications 1 à 8, de l'information représentative d'une quantité de substance active à vaporiser.

## Patentansprüche

1. Vorrichtung (10) zum Anpassen einer Wirkstoffmenge, die von einem Benutzer eingeatmet wird, Folgendes beinhaltend:
- zwei Behälter (105, 110), einen ersten Behälter (105), der eine Flüssigkeit beinhaltet, die eine Wirkstoffdichte aufweist, die geringer ist, als jene einer Flüssigkeit in dem zweiten Behälter (110), wobei jede Flüssigkeit konfiguriert ist, um zu verdampfen, wenn diese Flüssigkeit über eine vorbestimmte Temperaturgrenze hinaus erhitzt wird,
- ein Mittel (115) zum Einatmen des Dampfes einer verdampfenden Flüssigkeit aus jedem Behälter durch den Benutzer,
- zwei Heizwiderstände (120, 125), wobei jeder Behälter einem Heizwiderstand zugeordnet ist,
**dadurch gekennzeichnet, dass** sie beinhaltet:
- ein Mittel (130) zum Bestimmen einer Wirkstoffmenge zum Zerstäuben für ein vorbestimmtes Dampfvolumen,
- ein Mittel (135) zum Steuern der Erwärmung jedes Widerstands zum unabhängigen Betätigen eines jeden Widerstands in Abhängigkeit von der bestimmten zu zerstäubenden Wirkstoffmenge, wobei das Verhältnis der Erwärmung des Widerstands, der dem zweiten Behälter zugeordnet ist, zur Erwärmung des Widerstands, der dem ersten Behälter zugeordnet ist, eine steigende Funktion der bestimmten zu zerstäubenden Wirkstoffmenge für das vorbestimmte Dampfvolumen ist.

2. Vorrichtung (10) nach Anspruch 1, die ein Mittel (140) zum Zugreifen auf ein Benutzerprofil beinhaltet,
wobei das Mittel (130) zum Bestimmen einer zu zerstäubenden Wirkstoffmenge die Menge in Abhängigkeit von Daten des Benutzerprofils bestimmt.

3. Vorrichtung (10) nach einem der Ansprüche 1 oder 2, wobei das Mittel (130) zum Bestimmen eine zu zerstäubende Wirkstoffmenge in Abhängigkeit von einem Zeitstempeldatum bestimmt, das einer Inbetriebnahme der Vorrichtung zugeordnet ist.

4. Vorrichtung (10) nach Anspruch 3, wobei das Mittel (130) zum Bestimmen eine steigende Wirkstoffmenge im Verhältnis zur letzten bestimmten Wirkstoffmenge bestimmt, wenn das Zeitstempeldatum das erste Zeitstempeldatum größer als eine vorbestimmte Zeit ist.

5. Vorrichtung (10) nach Anspruch 3 oder 4, wobei das Mittel (130) zum Bestimmen eine im Allgemeinen sinkende Wirkstoffmenge in Abhängigkeit von dem Zeitstempeldatum bestimmt.

6. Vorrichtung (10) nach einem der Ansprüche 3 bis 5, die ein Mittel (145) zum Detektieren einer Einatmungsfrequenz des Benutzers an dem Mittel (115) zum Einatmen beinhaltet, wobei das Mittel (130) zum Bestimmen die Wirkstoffmenge in Abhängigkeit von der detektierten Einatmungsfrequenz bestimmt.

7. Vorrichtung (10) nach einem der Ansprüche 1 bis 6, die ein Mittel (150) zum Erfassen eines Blutalkoholgehalts des Benutzers beinhaltet, wobei das Mittel (130) zum Bestimmen die zu zerstäubende Wirkstoffmenge in Abhängigkeit von dem detektieren Blutalkoholgehalt bestimmt.

8. Vorrichtung (10) nach einem der Ansprüche 1 bis 7, die ein Mittel (155) zum Erfassen eines für eine Temperatur in mindestens einem Behälter (105, 110) repräsentativen Datums beinhaltet, wobei das Mittel (135) zum Steuern das Erwärmen des Widerstands (120, 125), der jedem Behälter zugeordnet ist, in Abhängigkeit von der erfassten Temperatur steuert.

9. Einheit, eine Vorrichtung nach einem der Ansprüche 1 und 8, und ein tragbares Kommunikationsendgerät (20) beinhaltend, **dadurch gekennzeichnet, dass** das Endgerät Folgendes beinhaltet:
- ein Mittel (205) zum Bestimmen einer für eine zu zerstäubende Wirkstoffmenge repräsentativen Information, und
- ein Mittel (210) zum Übertragen der für eine zu zerstäubende Wirkstoffmenge repräsentativen Information zu einer Vorrichtung (10) nach einem der Ansprüche 1 bis 8.

## Claims

1. Device (10) for adjusting an amount of an active substance inhaled by a user, comprising:
- two tanks (105, 110), a first tank (105) comprising a liquid having a lower density of active substance than a liquid in the second tank (110), each liquid being configured to evaporate when said liquid is heated beyond a predefined temperature limit;
- means (115) for inhaling, by the user, the liquid vapor evaporating from each tank;
- two heating resistors (120, 125), each tank being associated with one heating resistor;
**characterized in that** it comprises :
- means (130) for determining an amount of active substance to be vaporized for a predetermined volume of vapor;
- means (135) for controlling the heating of each resistor in order to actuate each resistor independently in accordance with the determined amount of active substance to be vaporized, the ratio of the heating of the resistor associated with the second tank to the heating of the resistor associated with the first tank being an increasing function of the determined amount of active substance to be vaporized for the predetermined volume of vapor.

2. Device (10) according to claim 1, which comprises means (140) for accessing a user profile,
the means (130) for determining an amount of active substance to be vaporized determining the amount according to data from the user profile.

3. Device (10) according to one of claims 1 or 2, wherein the determination means (130) determines an amount of active substance to be vaporized as a function of an item of timestamp data related to a starting up of the device.

4. Device (10) according to claim 3, wherein the determination means (130) determines an increasing amount of active substance, relative to the last amount of active substance determined, when the item of timestamp data is the first item of timestamp data greater than a predefined time.

5. Device (10) according to claim 3 or 4, wherein the determination means (130) determines a generally-decreasing amount of active substance as a function of the item of timestamp data.

6. Device (10) according to one of claims 3 to 5, which comprises means (145) for detecting the user's frequency of inhaling on the inhalation means (115), the determination means (130) determining the amount of active substance as a function of the inhalation frequency detected.

7. Device (10) according to one of claims 1 to 6, which comprises means (150) for capturing the user's blood-alcohol level; and the determination means (130) determining the amount of active substance to be vaporized as a function of the blood-alcohol level captured.

8. Device (10) according to one of claims 1 to 7, which comprises means (155) for capturing an item of data representative of a temperature in at least one tank, (105, 110), the control means (135) controls the heating of the resistor, (120, 125), associated with each said tank as a function of the temperature captured.

9. Communicating portable terminal (20), comprising:
- means (205) for determining an item of information representative of an amount of active substance to be vaporized; and
- means (210) for transmitting, to a device (10) according to one of claims 1 to 8, the item of information representative of an amount of active substance to be vaporized.
